# EUROPÄISCHE PATENTANMELDUNG

(11) **EP 1 374 681 A1**
(43) Veröffentlichungstag der Anmeldung: **02.01.2004**
(21) Anmeldenummer: 03013024.9
(22) Anmeldetag: 10.06.2003
(51) Int. Cl.: A01N 61/00, C12Q 1/533

(54) **Verfahren zum Identifizieren von fungizid wirksamen Verbindungen**

(30) Priorität: 18.06.2002 DE 10227001
(71) Anmelder: Bayer CropScience AG, 40789 Monheim (DE)
(72) Erfinder: Vaupel, Martin, Dr., 42799 Leichlingen (DE); Dunkel, Ralf, Dr., 40789 Monheim (DE); Kuck, Karl-Heinz, Dr., 40767 Langenfeld (DE)

(57) **Zusammenfassung**

Die Erfindung bezieht sich auf ein Verfahren zum Identifizieren von Fungiziden, die Verwendung einer Phosphomannomutase zum Identifizieren von Fungiziden, und die Verwendung von Inhibitoren der Phosphomannomutase als Fungizide.

## Beschreibung

Die Erfindung bezieht sich auf ein Verfahren zum Identifizieren von Fungiziden, die Verwendung von pilzlicher Phosphomannomutase zum Identifizieren von Fungiziden, und die Verwendung von Inhibitoren der Phosphomannomutase als Fungizide.

Unerwünschtes Pilzwachstum, das in der Landwirtschaft jedes Jahr zu beträchtlichen Schäden führt, kann durch die Verwendung von Fungiziden kontrolliert werden. Die Ansprüche an Fungizide sind dabei hinsichtlich ihrer Wirksamkeit, Kosten und vor allem ihrer Umweltverträglichkeit stetig angestiegen. Es existiert deshalb ein Bedarf an neuen Substanzen bzw. Substanzklassen, die zu leistungsfähigen und umweltverträglichen neuen Fungiziden entwickelt werden können. Im Allgemeinen ist es üblich, in Gewächshaustests nach solchen neuen Leitstrukturen zu suchen. Solche Tests sind jedoch arbeitsintensiv und teuer. Die Anzahl der Substanzen, die im Gewächshaus getestet werden können, ist entsprechend begrenzt. Eine Alternative zu solchen Tests ist die Verwendung von sogenannten Hochdurchsatzverfahren (HTS = high throughput screening). Dabei werden in einem automatisierten Verfahren eine große Anzahl von Einzelsubstanzen hinsichtlich ihrer Wirkung auf Zellen, individuelle Genprodukte oder Gene getestet. Wird für bestimmte Substanzen eine Wirkung nachgewiesen, so können diese in herkömmlichen Screeningverfahren untersucht und gegebenenfalls weiter entwickelt werden.

Vorteilhafte Angriffspunkte für Fungizide werden oft in essentiellen Biosynthesewegen gesucht. Ideale Fungizide sind weiterhin solche Stoffe, die Genprodukte hemmen, die eine entscheidende Bedeutung bei der Ausprägung der Pathogenität eines Pilzes haben.

Aufgabe der vorliegenden Erfindung war es deshalb, einen geeigneten neuen Angriffspunkt für potentielle fungizide Wirkstoffe zu identifizieren und zugänglich zu machen, und ein Verfahren zur Verfügung zu stellen, das die Identifizierung von Modulatoren dieses Angriffspunkts ermöglicht, die dann als Fungizide verwendet werden können.

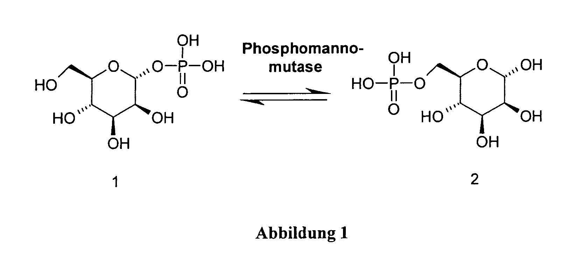

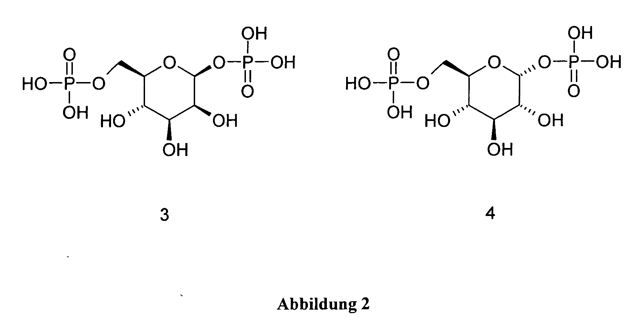

Die Phosphomannomutase (EC 5.4.2.8), auch bekannt als D-Mannose 1,6-phosphomutase, katalysiert die Isomerisierung von D-Mannose 1-phosphat zu D-Mannose 6-phosphat (Abbildung 1). Für diese Isomerisierung ist D-Mannose 1,6-bisphosphat bzw. D-Glucose 1,6-bisphosphat als Kofaktor notwendig (Abbildung 2). Über einen Phosphatgruppentransfer vom Kofaktor auf D-Mannose 1-phosphat entsteht als Zwischenprodukt D-Mannose 1,6-bisphosphat und durch die anschließende Abspaltung der Phosphatgruppe am C1-Atom das Endprodukt D-Mannose 6-phosphat und der recycelte Kofaktor (Oesterhelt et al., 1997).

Die Reaktion der Phosphomannomutase ist ein essentieller Schritt für die Bereitstellung von aktiviertem Zucker für N- und O-Glykosylierungen von Proteinen oder die Synthese von Glycosylphosphoinositol (GPI) Anker (Burda & Aebi 1999; Herscovics & Orlean, 1993; Hibbs et al.; 1988; Kepes, 1994).

Gene für die Phosphomannomutase wurden aus verschiedenen Organismen kloniert, z.B. aus *Saccharomyces cerevisiae* (Swissprot Accession No.: P07283). *Candida albicans* (Swissprot Accession No.: P31353), *Schizosaccharomyces pombe* (Swissprot Accession No.: AB000703), *Homo sapiens* (Swissprot: Accession No.: 015305) Maus (Swissprot: Accession No.: 035621) oder *Arabidopsis thaliana* (Swissprot Accession No.: 080840).

Neben den oben aufgeführten Organismen ist die Phosphomannomutase auch in Prokaryonten vorhanden, wie z.B. in *Pseudomonas aeruginosa* (EMBL Accession No.: M60873), *Azospirillum brasilense* (Swissprot Accession No. P45632) oder *Campylobacter jejuni* (EMBL Accession No.:AL139078)

Die Sequenzähnlichkeiten sind innerhalb der eukaryontischen Klassen bzw. innerhalb der bakteriellen Klasse signifikant, während hingegen die Sequenzidentität zwischen Vertretern der eukaryontischen Klasse und der bakteriellen Klasse nicht signifikant ist.

Die Phosphomannomutase wurde z.B. aus Hefe isoliert, teilweise gereinigt und auch teilweise charakterisiert (Kepes and Schekman, 1988; Glaser et al, 1966; Glaser et al., 1959).

Aufgabe der vorliegenden Erfindung war es deshalb ebenfalls, Phosphomannomutasen aus Pilzen sowie Verfahren zugänglich zu machen, in denen Inhibitoren des Enzyms identifiziert und auf ihre fungiziden Eigenschaften hin geprüft werden können.

Der Begriff "Identität", wie er hierin verwendet wird, bezieht sich auf die Zahl von Sequenzpositionen die in einem Alignment identisch sind. Sie wird meist in Prozent der Alignment Länge angegeben.

Der Begriff "Ähnlichkeit", wie er hierin verwendet wird, setzt dagegen die Definition einer Ähnlichkeitsmetrik voraus, also eines Maßes dafür, als wie ähnlich man beispielsweise ein Valin zu einem Threonin oder zu einem Leucin annehmen möchte.

Der Begriff "Homologie", wie er hierin verwendet wird, bedeutet wiederum evolutionäre Verwandtschaft. Zwei homologe Proteine haben sich aus einer gemeinsamen Vorläufersequenz entwickelt. Der Begriff hat nicht unbedingt etwas mit Identität oder Ähnlichkeit zu tun, abgesehen davon, dass homologe Sequenzen meist ähnlicher sind (oder in einem Alignment mehr identische Positionen besitzen) als nicht-homologe Sequenzen.

Der Ausdruck "vollständige Phosphomannomutase" wie er hierin verwendet wird, beschreibt die Phosphomannomutase, die von der vollständigen kodierenden Region einer Transkriptionseinheit kodiert wird, beginnend mit dem ATG-Startcodon und umfassend alle informationstragenden Exonbereiche des im Herkunftsorganismus vorliegenden, für Phosphomannomutase kodierenden Gens, sowie die für eine korrekte Termination der Transkription nötigen Signale.

Der Ausdruck "biologische Aktivität einer Phosphomannomutase" wie er hierin verwendet wird, bezieht sich auf die Fähigkeit eines Polypeptids, die vorstehend beschriebene Reaktion, d.h. die Umsetzung von D-Mannose 1-phosphat zu D-Mannose 6-phosphat zu katalysieren.

Der Ausdruck "aktives Fragment" wie er hierin verwendet wird, beschreibt nicht mehr vollständige Nukleinsäuren kodierend für Phosphomannomutase, die aber noch für Polypeptide mit der biologischen Aktivität einer Phosphomannomutase kodieren, und die eine für die Phosphomannomutase charakteristische Reaktion wie vorne beschrieben katalysieren können. Solche Fragmente sind kürzer als die oben beschriebenen vollständigen, für die Phosphomannomutase kodierenden Nukleinsäuren. Dabei können sowohl an den 3'- und/oder 5'-Enden der Sequenz Nukleinsäuren entfernt worden sein, es können aber auch Teile der Sequenz deletiert, d.h. entfernt worden sein, die die biologische Aktivität der Phosphomannomutase nicht entscheidend beeinträchtigen. Eine geringere oder gegebenenfalls auch eine erhöhte Aktivität, die aber noch die Charakterisierung bzw. Verwendung des resultierenden Phosphomannomutase Fragments gestattet, wird dabei als ausreichend im Sinne des hier verwendeten Ausdrucks verstanden. Der Ausdruck "aktives Fragment" kann sich ebenso auf die Aminosäuresequenz der Phosphomannomutase beziehen und gilt dann analog den obigen Ausführungen für solche Polypeptide, die im Vergleich zur oben definierten vollständigen Sequenz bestimmte Teile nicht mehr enthalten, wobei die biologische Aktivität des Enzyms jedoch nicht entscheidend beeinträchtigt ist. Die Fragmente können dabei verschiedene Länge besitzen.

Der Begriff "Phosphomannomutase-Hemmtest" oder "Hemmtest", wie er hierin verwendet wird, bezieht sich auf ein Verfahren bzw. einen Test, der es gestattet, die Inhibition der enzymatischen Aktivität eines Polypeptids mit der Aktivität einer Phosphomannomutase durch eine oder mehrere chemische Verbindungen (Kandidatenverbindung(en)) zu erkennen, wodurch die chemische Verbindung als Inhibitor der Phosphomannomutase identifiziert werden kann.

Der Ausdruck "Gen", wie er hierin verwendet wird, ist die Bezeichnung für einen Abschnitt aus dem Genom einer Zelle, die für die Synthese einer Polypeptid-Kette verantwortlich ist.

Der Ausdruck "Fungizid" bzw. "fungizid" wie er hierin verwendet wird, bezieht sich auf chemische Verbindungen, die zur Bekämpfung von Pilzen, insbesondere von pflanzenpathogenen Pilzen geeignet sind. Solche pflanzenpathogene Pilze werden nachfolgenden genannt, wobei die Aufzählung nicht abschließend ist:
Plasmodiophoromycetes, Oomycetes, Chytridiomycetes, Zygomycetes, Ascomycetes, Basidiomycetes und Deuteromycetes, z.B.

Pythium-Arten, wie beispielsweise *Pythium ultimum,* Phytophthora-Arten, wie beispielsweise *Phytophthora infestans,* Pseudoperonospora-Arten, wie beispielsweise *Pseudoperonospora humuli* oder *Pseudoperonospora cubensis,* Plasmopara-Arten, wie beispielsweise *Plasmopara viticola,* Bremia-Arten, wie beispielsweise *Bremia lactucae,* Peronospora-Arten, wie beispielsweise *Peronospora pisi* oder P. *brassicae,* Erysiphe-Arten, wie beispielsweise *Erysiphe graminis,* Sphaerotheca-Arten, wie beispielsweise *Sphaerotheca fuliginea,* Podosphaera-Arten, wie beispielsweise *Podosphaera leucotricha,* Venturia-Arten, wie beispielsweise *Venturia inaequalis,* Pyrenophora-Arten, wie beispielsweise *Pyrenophora teres* oder P. *graminea* (Konidienform: Drechslera, Syn: Helminthosporium), Cochliobolus-Arten, wie beispielsweise *Cochliobolus sativus* (Konidienform: Drechslera, Syn: Helminthosporium), Uromyces-Arten, wie beispielsweise *Uromyces appendiculatus,* Puccinia-Arten, wie beispielsweise *Puccinia recondita,* Sclerotinia-Arten, wie beispielsweise *Sclerotinia sclerotiorum,* Tilletia-Arten, wie beispielsweise *Tilletia caries;* Ustilago-Arten, wie beispielsweise *Ustilago nuda* oder *Ustilago avenae,* Pellicularia-Arten, wie beispielsweise *Pellicularia sasakii,* Pyricularia-Arten, wie beispielsweise *Pyricularia* *oryzae,* Fusarium-Arten, wie beispielsweise *Fusarium culmorum,* Botrytis-Arten, Septoria-Arten, wie beispielsweise *Septoria nodorum,* Leptosphaeria-Arten, wie beispielsweise *Leptosphaeria nodorum,* Cercospora-Arten, wie beispielsweise *Cercospora canescens,* Alternaria-Arten, wie beispielsweise *Alternaria brassicae* oder Pseudocercosporella-Arten, wie beispielsweise *Pseudocercosporella herpotrichoides.*

Von besonderem Interesse sind z.B. auch *Magnaporthe grisea, Cochliobulus heterostrophus, Nectria hematococcus* and Phytophtora species.

Fungizide Wirkstoffe, die mit Hilfe der erfindungsgemäßen Phosphomannomutase gefunden werden, können aber auch mit Phosphomannomutase aus humanpathogenen Pilzspezies interagieren, wobei die Interaktion mit den unterschiedlichen in diesen Pilzen vorkommenden Phosphomannomutasen nicht immer gleich stark sein muss.

Gegenstand der vorliegenden Erfindungen ist deshalb auch die Verwendung von Inhibitoren der Phosphomannomutase zum Herstellen von Mitteln zur Behandlung von durch humanpathogene Pilze hervorgerufenen Erkrankungen.

Dabei sind die folgenden humanpathogenen Pilze von besonderem Interesse, die die nachfolgend genannten Krankheitsbilder hervorrufen können:
Dermatophyten, wie z.B. Trichophyton spec., Microsporum spec., *Epidermophyton floccosum* oder *Keratomyces ajelloi,* die z.B. Fußmykosen (Tinea pedis) hervorrufen,
Hefen, wie z.B. *Candida albicans,* Soor-Ösophagitis und Dermatitis hervorruft, *Candida glabrata, Candida krusei* oder *Cryptococcus neoformans,* die z.B. pulmonale Cryptococcose und auch Torulose hervorrufen können,
Schimmelpilze, wie z.B. *Aspergillus fumigatus, A. flavus, A. niger,* die z.B. bronchopulmonale Aspergillose oder Pilzsepsis hervorrufen, Mucor spec., Absidia spec., oder Rhizopus spec., die z.B. Zygomykosen (intravasale Mykosen) hervorrufen, *Rhinosporidium seeberi,* der z.B. chronische granulomatöse Pharyngitis und Tracheitis hervorruft, *Madurella myzetomatis,* der z.B. subkutane Myzetome hervorruft, *Histoplasma capsulatum,* der z.B. retikuloendotheliale Zytomykose und M. Darling hervorruft, *Coccidioides immitis,* der z. B. pulmonale Coccidioidomykose und Sepsis hervorruft, *Paracoccidioides brasiliensis,* der z.B. brasilianische Blastomykose hervorruft, *Blastomyces dermatitidis,* der z.B. Gilchrist-Krankheit und nordamerikanische Blastomykose hervorruft, *Loboa loboi,* der z.B. Keloid-Blastomykose und Lobo's Krankheit hervorruft, und *Sporothrix schenckii,* der z.B. Sporotrichose (granulomatöse Hautmykose) hervorruft.

Fungizide Wirkstoffe, die mit Hilfe einer aus einem bestimmten Pilz, hier aus S. *cerevisiae,* gewonnenen Phosphomannomutase gefunden werden, können also auch mit Phosphomannomutase aus anderen zahlreichen Pilzspezies, gerade auch mit pflanzenpathogenen Pilzen interagieren, wobei die Interaktion mit den unterschiedlichen in diesen Pilzen vorkommenden Phosphomannomutasen nicht immer gleich stark sein muss. Dies erklärt unter anderem die beobachtete Selektivität der an diesem Enzym wirksamen Substanzen.

Der Ausdruck "heterologer Promotor", wie er hierin verwendet wird, bezieht sich auf einen Promotor, der andere Eigenschaften als derjenige Promotor aufweist, der im Ursprungsorganismus die Expression des betreffenden Gens kontrolliert.

Der Ausdruck "Kompetitor" wie er hierin verwendet wird, bezieht sich auf die Eigenschaft der Verbindungen, mit anderen, gegebenenfalls zu identifizierenden Verbindungen um die Bindung an der Phosphomannomutase zu kompetitieren und diese vom Enzym zu verdrängen bzw. von diesen verdrängt zu werden.

Der Ausdruck "Agonist", wie er hierin verwendet wird, bezieht sich auf ein Molekül, das die Aktivität der Phosphomannomutase beschleunigt oder verstärkt.

Der Ausdruck "Antagonist", wie er hierin verwendet wird, bezieht sich auf ein Molekül, das die Aktivität der Phosphomannomutase verlangsamt oder verhindert.

Der Ausdruck "Modulator", wie er hierin verwendet wird, stellt den Oberbegriff zu Agonist bzw. Antagonist dar. Modulatoren können kleine organisch-chemische Moleküle, Peptide oder Antikörper sein, die an die erfindungsgemäßen Polypeptide binden bzw. die deren Aktivität beeinflussen. Weiterhin können Modulatoren kleine organisch-chemische Moleküle, Peptide oder Antikörper sein, die an ein Molekül binden, welches wiederum an die erfindungsgemäßen Polypeptide bindet, und dadurch deren biologische Aktivität beeinflusst. Modulatoren können natürliche Substrate und Liganden darstellen oder strukturelle oder funktionelle Mimetika davon. Bevorzugt handelt es sich beim Ausdruck "Modulator", wie er hierin verwendet wird, jedoch um solche Moleküle, die nicht die natürlichen Substrate bzw. Liganden darstellen.

Trotz der umfangreichen Forschung an der Phosphomannomutase war bislang unbekannt, dass die Phosphomannomutase in Pilzen ein Zielprotein (ein so genanntes "Target") fungizid wirksamer Substanzen sein kann. Bislang bekannte Inhibitoren der Phosphomannomutase, bei denen es sich um Substratanaloga handelt (Martin et al., 1999; Guha & Rose, 1985), haben keine bekannte fungizide Wirkung. Damit wird in der vorliegenden Erfindung zum ersten Mal gezeigt, dass die Phosphomannomutase ein insbesondere für Pilze wichtiges Enzym darstellt und deshalb in besonderem Maße dazu geeignet ist, als Zielprotein für die Suche nach weiteren und verbesserten fungizid wirksamen Wirkstoffen verwendet zu werden.

Im Rahmen der vorliegenden Erfindung wurde also gerunden, dass die Phosphomannomutase ein Angriffspunkt oder "Target" für fungizide Wirkstoffe sein kann, die Inhibition der Phosphomannomutase also zur Schädigung oder zum Absterben des Pilzes führt. Das Enzym Phosphomannomutase wurde weiter als ein Polypeptid erkannt, das zum Identifizieren von Modulatoren bzw. Inhibitoren seiner enzymatischen Aktivität in geeigneten Testverfahren verwendet werden kann, was bei verschiedenen theoretisch interessanten Targets nicht selbstverständlich gegeben ist.

Im Rahmen der vorliegenden Erfindung wurde deshalb ein Verfahren entwickelt, das geeignet ist, die Aktivität der Phosphomannomutase sowie gegebenenfalls die Hemmung dieser Aktivität bei Anwesenheit eines potentiellen Inhibitors der Phosphomannomutase in einem so genannten Hemmtest zu bestimmen, auf diese Weise Inhibitoren des Enzyms z.B. in HTS- und UHTS-Verfahren zu identifizieren und deren fungizide Eigenschaften zu prüfen. Im Rahmen der vorliegenden Erfindung wurde so auch gezeigt, dass die Inhibitoren der Phosphomannomutase aus Pilzen als Fungizide verwendet werden können.

Im Rahmen der vorliegenden Erfindung wurde weiter gefunden, dass die Phosphomannomutase auch *in vivo* durch Wirkstoffe inhibiert werden kann und ein mit diesen Wirkstoffen behandelter pilzlicher Organismus durch die Behandlung mit diesen Wirkstoffen geschädigt und abgetötet werden kann. Die Inhibitoren einer pilzlichen Phosphomannomutase können also als Fungizide im Pflanzenschutz oder als Antimykotika in Pharmaindikationen verwendet werden. In der vorliegenden Erfindung wird beispielsweise gezeigt, dass die Hemmung der Phosphomannomutase mit einer in einem erfindungsgemäßen Verfahren identifizierten Substanz zur Schädigung oder zum Absterben der behandelten Pilze in synthetischen Medien bzw. auf der Pflanze führt.

Das Enzym Phosphomannomutase kann z.B. aus Pilzen wie *S. cerevisiae* gewonnen werden. Zur Herstellung der Phosphomannomutase aus Hefe kann das Gen z.B. rekombinant in *Escherichia coli* exprimiert und aus *E. coli* Zellen eine Enzympräparation hergestellt werden (Beispiel 1).

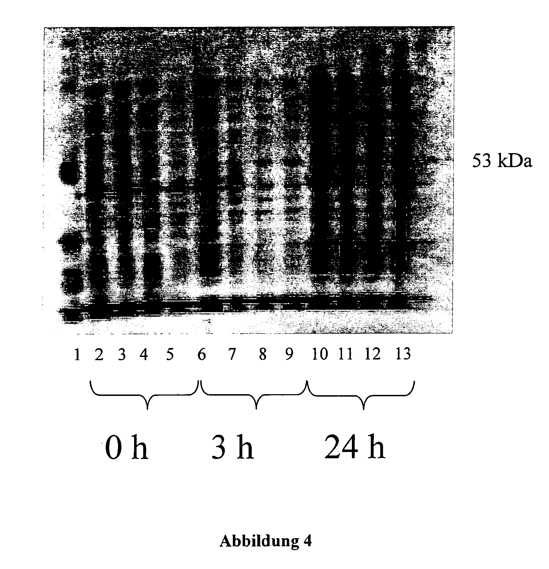

So wurde für die Expression des von *sec53* kodierten Polypeptids Sec53 (Bernstein et al., 1985; SWISS-PROT Accession Nummer: P07283) der zugehörige ORF aus genomischer DNA nach dem Fachmann bekannten Methoden über genspezifische Primer amplifiziert. Die entsprechende DNA wurde in den Vektor pGEX-4T-1 (Pharmacia Biotech, ermöglicht die Einführung eines N- terminalen GST-Tags) kloniert. Das resultierende Plasmid pSec53 enthält die vollständige kodierende Sequenz von *sec53* in einer N-terminalen Fusion mit einem GST-Tag aus dem Vektor. Das Sec53 Fusionsprotein besitzt eine berechnete Masse von 53 kD (Beispiel 1 und Abbildung 4).

Das Plasmid pSec53 wurde dann zur rekombinanten Expression von Sec53 in *E. coli* BL21(DE3) Zellen verwendet (Beispiel 1).

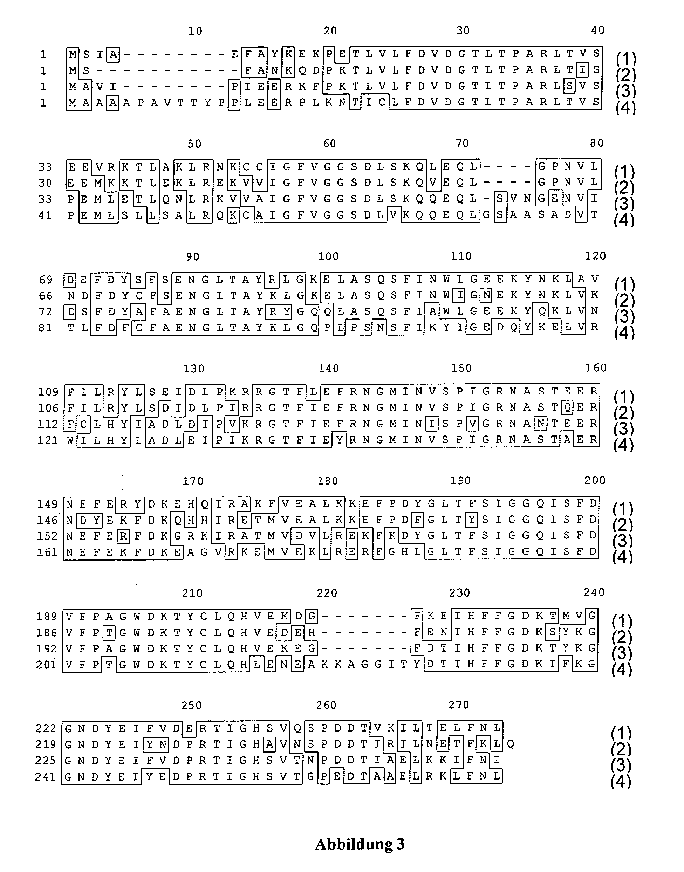

Wie bereits vorstehend ausgeführt, ist die vorliegende Erfindung nicht nur auf die Verwendung von Phosphomannomutase aus Hefe beschränkt. In analoger und dem Fachmann bekannter Weise können auch aus anderen Pilzen, vorzugsweise aus pflanzenpathogenen Pilzen, Polypeptide mit der Aktivität einer Phosphomannomutase gewonnen werden, die dann in einem erfindungsgemäßen Verfahren eingesetzt werden können. Diese Möglichkeit wird u.a. durch die hohe Homologie zwischen Phosphomannomutasen aus verschiedenen Pilzspezies eröffnet (vgl. Abb. 3). Bevorzugt wird die Phosphomannomutase aus S. *cerevisiae* verwendet.

Der Ausdruck "Polypeptide", wie er hierin verwendet wird, bezieht sich sowohl auf kurze Aminosäureketten, die gewöhnlich als Peptide, Oligopeptide oder Oligomere bezeichnet werden, als auch auf längere Aminosäureketten, die gewöhnlich als Proteine bezeichnet werden. Er umfasst Aminosäureketten, die entweder durch natürliche Prozesse, wie posttranslationale Prozessierung, oder durch chemische Verfahren, die Stand der Technik sind, modifiziert sein können. Solche Modifikationen können an verschiedenen Stellen und mehrfach in einem Polypeptid vorkommen, wie beispielsweise am Peptid-Rückgrat, an der Aminosäure-Seitenkette, am Aminound/oder am Carboxy-Terminus. Sie umfassen beispielsweise Acetylierungen, Acylierungen, ADP-Ribosylierungen, Amidierungen, kovalente Verknüpfungen mit Flavinen, Häm-Anteilen, Nukleotiden oder Nukleotid-Derivaten, Lipiden oder Lipid-Derivaten oder Phophatidylinositol, Cyclisierungen, Disulfidbrückenbildungen, Demethylierungen, Cystin-Bildungen, Formylierungen, gamma-Carboxylierungen, Glycosylierungen, Hydroxylierungen, Iodierungen, Methylierungen, Myristoylierungen, Oxidationen, proteolytische Prozessierungen, Phosphorylierungen, Selenoylierungen und tRNA-vermittelte Additionen von Aminosäuren.

Die erfindungsgemäßen Polypeptide können in der Form "reifer" Proteine oder als Teile größerer Proteine, z.B. als Fusionsproteine, vorliegen. Weiterhin können sie Sezernierungs- oder "Leader"-Sequenzen, Pro-Sequenzen, Sequenzen, die eine einfache Reinigung ermöglichen, wie mehrfache Histidin-Reste, oder zusätzliche stabilisierende Aminosäuren aufweisen. Die erfindungsgemäßen Proteine können ebenfalls so vorliegen, wie sie natürlicherweise in ihrem Herkunftsorganismus vorliegen, aus dem sie zum Beispiel direkt gewonnen werden können. In den erfindungsgemäßen Verfahren können ebenso aktive Fragmente einer Phosphomannomutase eingesetzt werden, solange sie die Bestimmung der enzymatischen Aktivität des Polypeptids bzw. deren Inhibition durch eine Kandidatenverbindung ermöglichen.

Die in den erfindungsgemäßen Verfahren eingesetzten Polypeptide können im Vergleich zu den entsprechenden Regionen von natürlich vorkommenden Phosphomannomutasen Deletionen oder Aminosäuresubstitutionen aufweisen, solange sie zumindest noch die biologische Aktivität einer vollständigen Phosphomannomutase zeigen. Konservative Substitutionen sind bevorzugt. Solche konservativen Substitutionen umfassen Variationen, wobei eine Aminosäure durch eine andere Aminosäure aus der folgenden Gruppe ersetzt wird:
1. Kleine aliphatische, nicht-polare oder wenig polare Reste: Ala, Ser, Thr, Pro und Gly;
2. Polare, negativ geladene Reste und deren Amide: Asp, Asn, Glu und Gln;
3. Polare, positiv geladene Reste: His, Arg und Lys;
4. Große aliphatische, nicht-polare Reste: Met, Leu, Ile, Val und Cys; und
5. Aromatische Reste: Phe, Tyr und Trp.

Ein mögliches Reinigungsverfahren der Phosphomannomutase basiert auf präparativer Elektrophorese, FPLC, HPLC (z.B. unter Anwendung von Gelfiltrations-, Reversphasen- oder leicht hydrophoben Säulen), Gelfiltration, differentieller Präzipitation, Ionenaustausch-Chromatographie oder Affinitätschromatographie (vgl. Beispiel 1).

Ein schnelles Verfahren zum Isolieren von Phosphomannomutasen, die von Wirtszellen synthetisiert werden, beginnt mit der Expression eines Fusionsproteins, wobei der Fusionspartner auf einfache Weise affinitätsgereinigt werden kann. Der Fusionspartner kann beispielsweise ein GST-Tag sein (vgl. Beispiel 1). Das Fusionsprotein kann dann an einer Glutathion-Sepharosesäule gereinigt werden. Der Fusionspartner kann durch partielle proteolytische Spaltung beispielsweise an Linkern zwischen dem Fusionspartner und dem zu reinigenden erfindungsgemäßen Polypeptid abgetrennt werden. Der Linker kann so gestaltet werden, dass er Ziel-Aminosäuren, wie Arginin- und Lysin-Reste einschließt, die Stellen für eine Spaltung durch Trypsin definieren. Um solche Linker zu erzeugen, können Standard-Klonierungsverfahren unter Verwendung von Oligonukleotiden angewendet werden.

Weitere mögliche Reinigungsverfahren basieren wiederum auf präparativer Elektrophorese, FPLC, HPLC (z.B. unter Anwendung von Gelfiltrations-, Reversphasenoder leicht hydrophoben Säulen), Gelfiltration, differentieller Präzipitation, Ionenaustausch-Chromatographie und Affinitätschromatographie.

Die Ausdrücke "Isolierung oder Reinigung", wie sie hierin verwendet werden, bedeuten, dass die erfindungsgemäßen Polypeptide von anderen Proteinen oder anderen Makromolekülen der Zelle oder des Gewebes abgetrennt werden. Vorzugsweise ist eine die erfindungsgemäßen Polypeptide enthaltende Zusammensetzung hinsichtlich des Proteingehalts gegenüber einer Präparation aus den Wirtszellen mindestens 10-fach und besonders bevorzugt mindestens 100-fach angereichert.

Die erfindungsgemäßen Polypeptide können auch ohne Fusionspartner mit Hilfe von Antikörpern, die an die Polypeptide binden, affinitätsgereinigt werden.

Das Verfahren zum Herstellen von Polypeptiden mit der Aktivität einer Phosphomannomutase, wie z.B. des Polypeptids Sec53, ist damit gekennzeichnet durch
(a) das Kultivieren einer Wirtszelle enthaltend zumindest eine exprimierbare Nukleinsäuresequenz kodierend für ein Polypeptid aus Pilzen mit der biologischen Aktivität einer Phosphomannomutase unter Bedingungen, die die Expression dieser Nukleinsäure gewährleisten, oder
(b) das Exprimieren einer exprimierbaren Nukleinsäuresequenz kodierend für ein Polypeptid aus Pilzen mit der biologischen Aktivität einer Phosphomannomutase in einem *in vitro*-System, und
(c) die Gewinnung des Polypeptids aus der Zelle, dem Kulturmedium oder dem *in* vitro-System.

Die so erhaltenen Zellen enthaltend das erfindungsgemäße Polypeptid oder das so erhaltene gereinigte Polypeptid sind geeignet, in Verfahren zum Identifizieren von Modulatoren bzw. Inhibitoren der Phosphomannomutase verwendet zu werden.

Gegenstand der vorliegenden Erfindung ist auch die Verwendung von Polypeptiden aus Pilzen, welche zumindest eine biologische Aktivität einer Phosphomannomutase ausüben, in Verfahren zum Identifizieren von Inhibitoren eines Polypeptids aus Pilzen mit der Aktivität einer Phosphomannomutase, wobei die Inhibitoren der Phosphomannomutase als Fungizide verwendet werden können. Besonders bevorzugt wird in diesen Verfahren die Phosphomannomutase aus *S. cerevisiae* verwendet.

Fungizide Wirkstoffe, die mit Hilfe einer Phosphomannomutase aus einer bestimmten Pilzspezies gefunden werden, können auch mit Phosphomannomutasen aus anderen Pilzspezies interagieren, wobei die Interaktion mit den unterschiedlichen in diesen Pilzen vorkommenden Phosphomannomutasen nicht immer gleich stark sein muss. Dies erklärt unter anderem die Selektivität wirksamer Substanzen. Die Nutzung der Wirkstoffe, die mit einer spezifischen Phosphomannomutase gefunden wurden, als Fungizid auch bei anderen Pilzen kann darauf zurückgeführt werden, dass sich Phosphomannomutasen aus verschiedenen Pilzspezies sehr nahe stehen und in größeren Bereichen eine ausgeprägte Homologie zeigen. So wird an Abbildung 3 deutlich, dass eine solche Homologie über beträchtliche Sequenzabschnitte hinweg zwischen *S. cerevisiae, C. albicans, S. pombe* und *N. crassa* besteht und damit die Wirkung der mit Hilfe der Phosphomannomutase aus Hefe gefundenen Substanzen nicht auf *S. cerevisiae* beschränkt bleibt.

Gegenstand der vorliegenden Erfindung ist deshalb auch ein Verfahren zum Identifizieren von Fungiziden durch Testen von potentiellen Inhibitoren bzw. Modulatoren der enzymatischen Aktivität der Phosphomannomutase (Kandidatenverbindung) in einem Phosphomannomutase-Hemmtest.

Verfahren, die geeignet sind, Modulatoren, insbesondere Inhibitoren bzw. Antagonisten der erfindungsgemäßen Polypeptide zu identifizieren, beruhen in aller Regel auf der Bestimmung der Aktivität bzw. der biologischen Funktionalität des Polypeptids. Dazu kommen prinzipiell sowohl auf ganzen Zellen beruhende Verfahren *(in vivo* Verfahren) in Frage, wie auch Verfahren, die auf der Verwendung des aus den Zellen isolierten Polypeptids beruhen, das in gereinigter oder teilweise gereinigter Form oder auch als Rohextrakt vorliegen kann. Diese zellfreien *in vitro* Verfahren können ebenso wie *in vivo* Verfahren im Labormaßstab, in bevorzugter Weise aber auch in HTS oder UHTS Verfahren genutzt werden. Im Anschluss an die *in vivo* oder *in vitro* Identifizierung von Modulatoren des Polypeptids können Tests an Pilzkulturen durchgeführt werden, um die fungizide Wirksamkeit der gefundenen Verbindungen zu prüfen.

Viele Testsysteme, die die Prüfung von Verbindungen und natürlichen Extrakten zum Ziel haben, sind bevorzugt auf hohe Durchsatzzahlen ausgerichtet, um die Zahl der untersuchten Substanzen in einem gegebenen Zeitraum zu maximieren. Testsysteme, die auf zellfreiem Arbeiten beruhen, brauchen gereinigtes oder semigereinigtes Protein. Sie sind in erste Linie geeignet für eine "erste" Prüfung, die in erster Linie darauf abzielt, einen möglichen Einfluss einer Substanz auf das Zielprotein zu detektieren. Ist eine solche erste Prüfung erfolgt und eine oder mehrere Verbindungen, Extrakte etc. gefunden, kann die Wirkung solcher Verbindungen im Labor noch gezielter untersucht werden. So kann in einem ersten Schritt die Inhibierung oder Aktivierung des erfindungsgemäßen Polypeptids *in vitro* noch einmal geprüft werden, um im Anschluss daran die Wirksamkeit der Verbindung am Zielorganismus, hier einem oder mehreren pflanzenpathogenen Pilzen, zu testen. Die Verbindung kann dann gegebenenfalls als Ausgangspunkt für die weitere Suche und Entwicklung von fungiziden Verbindungen verwendet werden, die auf der ursprünglichen Struktur basieren, jedoch z.B. hinsichtlich Wirksamkeit, Toxizität oder Selektivität optimiert sind.

Um Modulatoren aufzufinden, kann z.B. ein synthetischer Reaktionsmix (z.B. Produkte der *in vitro* Transkription) oder ein zellulärer Bestandteil, wie eine Membran, ein Kompartiment oder irgendeine andere Präparation, die die erfindungsgemäßen Polypeptide enthält, zusammen mit einem gegebenenfalls markierten Substrat, Kofaktor oder Liganden der Polypeptide in Gegenwart und Abwesenheit eines Kandidatenmoleküls, das ein Antagonist sein kann, inkubiert werden. Die Fähigkeit des Kandidatenmoleküls die Aktivität der erfindungsgemäßen Polypeptide zu hemmen, wird z.B. erkennbar an einer verringerten Bindung des gegebenenfalls markierten Liganden oder an einer verringerten Umsetzung des gegebenenfalls markierten Substrates. Moleküle, die die biologische Aktivität der erfindungsgemäßen Polypeptide hemmen, sind gute Antagonisten.

Die Detektion der biologischen Aktivität der erfmdungsgemäßen Polypeptide kann durch ein so genanntes Reportersystem verbessert werden. Reportersysteme in dieser Hinsicht umfassen, sind aber nicht beschränkt auf colorimetrisch oder fluorimetrisch nachweisbare Substrate, die in ein Produkt umgewandelt werden oder ein Reportergen, das auf Veränderungen der Aktivität oder der Expression der erfindungsgemäßen Polypeptide anspricht oder andere bekannte Bindungstests.

Ein weiteres Beispiel für ein Verfahren, mit welchem Modulatoren der erfindungsgemäßen Polypeptide aufgefunden werden können, ist ein Verdrängungstest, bei dem man unter dafür geeigneten Bedingungen die erfindungsgemäßen Polypeptide und einen potenziellen Modulator mit einem Molekül, das bekanntermaßen an die erfindungsgemäßen Polypeptide bindet, wie einem natürlichen Substrat oder Liganden oder einem Substrat- oder Liganden-Mimetikum zusammenbringt. Die erfindungsgemäßen Polypeptide selbst können markiert werden, z.B. fluorimetrisch oder colorimetrisch, so dass man die Anzahl der Polypeptide, die an einen Liganden gebunden sind oder die eine Umsetzung mitgemacht haben, exakt bestimmen kann. Ebenso kann jedoch die Bindung mittels des gegebenenfalls markierten Substrats, Liganden bzw. Substratanalogen verfolgt werden. Auf diese Weise lässt sich die Effektivität des Antagonisten ermessen.

Effekte wie Zelltoxizität werden in diesen *in vitro* Systemen in der Regel ignoriert. Die Testsysteme überprüfen sowohl inhibierende bzw. suppressive Effekte der Substanzen als auch stimulatorische Effekte. Die Effektivität einer Substanz kann durch konzentrationsabhängige Testreihen überprüft werden. Kontrollansätze ohne Testsubstanzen bzw. ohne Enzym können zur Bewertung der Effekte herangezogen werden.

Durch die anhand der vorliegenden Erfindung verfügbaren, für eine erfindungsgemäße Phosphomannomutase kodierende Nukleinsäuren enthaltenden Wirtszellen, wird die Entwicklung von Testsystemen, die auf Zellen basieren, zur Identifizierung von Substanzen ermöglicht, die die Aktivität der erfindungsgemäßen Polypeptide modulieren.

Eine andere Möglichkeit zur Identifizierung von Substanzen, die die Aktivtät der erfindungsgemäßen Polypeptide modulieren, ist so auch der sogenannten "Scintillation Proximity Assay" (SPA), siehe EP 015 473. Dieses Testsystem nutzt die Interaktion eines Polypeptids (z.B. Phosphomannomutase aus Hefe) mit einem radiomarkierten Liganden bzw. Substrat. Das Polypeptid ist dabei an kleine Kügelchen ("Microspheres") oder Perlen ("Beads") gebunden, die mit szintillierenden Molekülen versehen sind. Im Verlauf des Abfalls der Radioaktivität wird die szintillierende Substanz im Kügelchen durch die subatomaren Partikel des radioaktiven Markers angeregt und ein detektierbares Photon emittiert. Die Testbedingungen werden so optimiert, dass nur jene vom Liganden ausgehenden Partikel zu einem Signal führen, die von einem an das erfindungsgemäße Polypeptid gebundenen Liganden ausgehen.

Vorzugsweise handelt es sich bei den zu identifizierenden Modulatoren um kleine organisch-chemische Verbindungen.

Ein Verfahren zum Identifizieren einer Verbindung, die die Aktivität einer Phosphomannomutase aus Pilzen moduliert und die als Fungizid im Pflanzenschutz verwendet werden kann, besteht bevorzugt darin, dass man
a) ein erfindungsgemäßes Polypeptid oder eine Wirtszelle enthaltend dieses Polypeptid mit einer chemischen Verbindung oder mit einem Gemisch von chemischen Verbindungen unter Bedingungen in Kontakt bringt, die die Interaktion der chemischen Verbindung mit dem Polypeptid erlauben,
b) die Aktivität des erfindungsgemäßen Polypeptids bei Abwesenheit einer chemischen Verbindung mit der Aktivität des erfindungsgemäßen Polypeptids bei Anwesenheit einer chemischen Verbindung oder eines Gemisches von chemischen Verbindungen vergleicht, und
c) die chemische Verbindung bestimmt, die die Aktivität des erfindungsgemäßen Polypeptids spezifisch moduliert und gegebenenfalls
d) die fungizide Wirkung der bestimmten Verbindung *in vivo* prüft.

Besonders bevorzugt wird dabei diejenige Verbindung bestimmt, die die Aktivität des erfindungsgemäßen Polypeptids spezifisch inhibiert. Der Begriff "Aktivität", wie er hier verwendet wird, bezieht sich auf die biologische Aktivität des erfindungsgemäßen Polypeptids.

Ein bevorzugtes Verfahren nutzt die Tatsache aus, dass die Phosphat-Gruppe von D-Mannose-6-phosphat säurestabil ist, während die Phosphat-Gruppe von D-Mannose-1-phosphat säurelabil ist. Die Aktivität bzw. die Ab- oder Zunahme der Aktivität des erfindungsgemäßen Polypeptids kann deswegen mittels Nachweis der durch Säure abgespalteten Phosphatgruppe von D-Mannose-1-Phosphat mit einem Phosphatnachweisreagenz bestimmt werden. Dabei wird die geringere bzw. inhibierte Aktivität des erfindungsgemäßen Polypeptids anhand der photospektrometrischen Bestimmung der Abnahme bzw. der Zunahme der durch Säure abspaltbaren Phosphatgruppe von D-Mannose-1-P verfolgt. Die Konzentration des freigesetzten Phosphats kann dann mit einem Phosphatnachweisreagenz bei einem Absorptionsmaximum bei 620 nm bestimmt werden.

Die Messung kann auch in für HTS- oder UHTS-Assays gängigeren Formaten erfolgen, z.B. in Mikrotiterplatten, in denen z.B. ein Gesamtvolumen von 5 bis 50 µl pro Ansatz bzw. pro Well vorgelegt wird und die einzelnen Komponenten in einer der vorstehend angegebenen Endkonzentrationen vorliegen (vgl. Beispiel 2). Dabei wird die zu testende, potentiell die Aktivität des Enzyms inhibierende oder aktivierende Verbindung (Kandidatenmolekül) z.B. in einer geeigneten Konzentration im oben angegebenen Testpuffer enthaltend D-Mannose-1-phosphat vorgelegt. Dann wird das erfindungsgemäße Polypeptid in oben genanntem Testpuffer, enthaltend NADP⁺, Glucose und die für den gekoppelten Assay notwendigen Hilfsenzyme Phosphoglucose Isomerase und Glucose-6-phosphat Dehydrogenase zugegeben und die Reaktion damit gestartet. Der Ansatz wird dann z.B. bis zu 2 oder 3 Stunden bei einer geeigneten Temperatur inkubiert und die Fluoreszenzzunahme bei einer Anregungswellenlänge von 360 nm und einer Emissionswellenlänge von 460 nm gemessen.

Eine weitere Messung erfolgt in einem entsprechenden Ansatz, jedoch ohne Zugabe eines Kandidatenmoleküls und ohne Zugabe eines erfindungsgemäßen Polypeptids (Negativkontrolle). Eine weitere Messung erfolgt wiederum bei Abwesenheit eines Kandidatenmoleküls, jedoch bei Anwesenheit des erfindungsgemäßen Polypeptids (Positivkontrolle). Negativ- und Positivkontrolle ergeben damit die Vergleichswerte zu den Ansätzen bei Anwesenheit eines Kandidatenmoleküls.

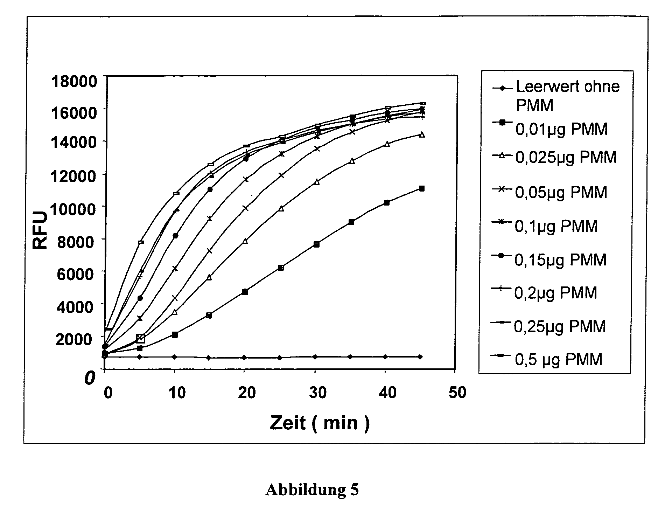

Um optimale Bedingungen für ein Verfahren zum Identifizieren von Inhibitoren der Phosphomannomutase bzw. zur Bestimmung der Aktivität der erfindungsgemäßen Polypeptide zu ermitteln, kann es vorteilhaft sein, den jeweiligen K_{M}-Wert des verwendeten erfindungsgemäßen Polypeptids zu bestimmen. Dieser gibt Aufschluss über die bevorzugt zu verwendende Konzentration des bzw. der Substrate. Im Fall der Phosphomannomutase aus Hefe konnte ein K_{M} von 0,22 mM für D-Mannose-1-phosphat bestimmt werden (Abbildung 5).

Mit Hilfe der vorstehend beispielhaft beschriebenen Verfahren konnten Verbindungen identifiziert werden, die die pilzliche Phosphomannomutase inhibieren.

Es versteht sich von selbst, dass neben den genannten Verfahren zur Bestimmung der enzymatischen Aktivität einer Phosphomannomutase bzw. der Inhibition dieser Aktivität und zum Identifizieren von Fungiziden auch andere, z.B. bereits bekannte, Verfahren bzw. Hemmtests verwendet werden können, solange diese Verfahren es gestatten, die Aktivität einer Phosphomannomutase zu bestimmen und eine Inhibition dieser Aktivität durch eine Kandidatenverbindung zu erkennen.

In Tabelle I werden beispielhaft Verbindungen gezeigt, die mit einem erfindungsgemäßen Verfahren als Inhibitoren der Phosphomannomutase identifiziert werden konnten.

Der dort angegebene pI50-Wert ist der negative dekadische Logarithmus des so genannten IC50-Werts, der die molare Konzentration einer Substanz angibt, die zur 50 %igen Hemmung des Enzyms führt.

Ein pI50-Wert von 8 entspricht z.B. einer halbmaximalen Hemmung des Enzyms bei einer Konzentration von 10 nM.

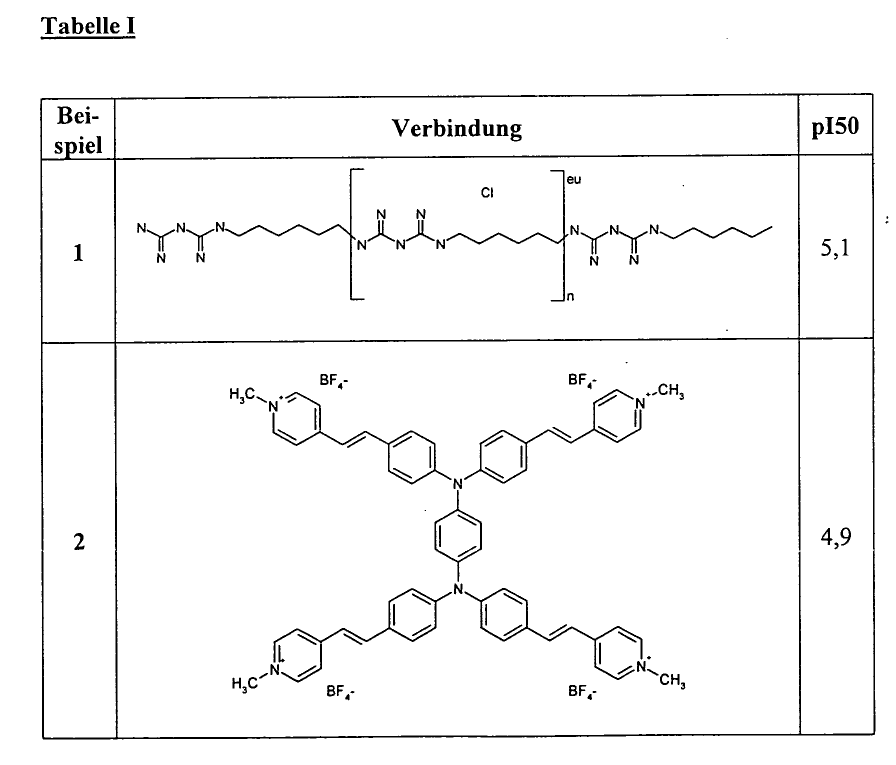

Im Rahmen der vorliegenden Erfindung konnte weiter gezeigt werden, dass die mit Hilfe eines erfindungsgemäßen Verfahrens identifizierten Inhibitoren einer erfindungsgemäßen Phosphomannomutase geeignet sind, Pilze zu schädigen oder zu töten.

Dazu können z.B. in die Kavitäten von Mikrotiterplatten eine Lösung des zu prüfenden Wirkstoffs pipettiert werden. Nachdem das Lösungsmittel abgedampft war, wird zu jeder Kavität Medium hinzugefügt. Das Medium wird vorher mit einer geeigneten Konzentration von Sporen bzw. Mycel des zu prüfenden Pilzes versetzt. Die resultierenden Konzentrationen des Wirkstoffes betragen z.B. 0,1, 1, 10 und 100 ppm.

Die Platten werden anschließend auf einem Schüttler bei einer Temperatur von 22°C inkubiert, bis in der unbehandelten Kontrolle ein ausreichendes Wachstum feststellbar ist.

Die Auswertung erfolgt dann photometrisch bei einer Wellenlänge von 620 nm. Aus den Messdaten der verschiedenen Konzentrationen kann die Wirkstoffdosis bestimmt werden, die zu einer 50 %igen Hemmung des Pilzwachstums gegenüber der unbehandelten Kontrolle führt (ED₅₀).

Ein anderes Verfahren zur Prüfung der fungiziden Wirkung beruht darauf, die protektive Wirkung der zu testenden Verbindung an einer Pflanze zu testen. Dazu wird die Pflanze mit einer Lösung des zu prüfenden Wirkstoffs besprüht und anschließend mit einer Lösung von Pilzssporen inokuliert und über mehrere Tage hinweg beobachtet. Es kann dann im Vergleich zu einer Pflanze, die nicht vorab mit einer Wirkstofflösung behandelt wurde, festgestellt werden, ob der zu prüfende Wirkstoff den Befall bzw. die Ausbreitung des Pilzes verhindert oder mindert.

Auch hier kann die Wirkstoffkonzentration bestimmt werden, die zu einer 50 %igen Hemmung des Pilzwachstums gegenüber der unbehandelten Kontrolle führt (ED₅₀).

Weiterhin kann als Größe zur Beurteilung einer fungiziden Wirkung der Wirkungsgrad herangezogen werden, wobei 0 % für einen Wirkungsgrad steht, der demjenigen der Kontrolle entspricht, während ein Wirkungsgrad von 100 % bedeutet, daß kein Befall beobachtet wird.

In Tabelle II sind die Ergebnisse eines solchen Tests als ED₅₀-Wert sowie als Wirkungsgrad (WG) für eine in einem erfindungsgemäßen Verfahren gefundene Verbindung (Tab. I, Bsp. 2) beispielhaft wiedergegeben.

**Tabelle II**

| **Organismus** | **ED**_{**50**} **[ppm]** | **WG [%]** |
|---|---|---|
| *Phytophtora infectans* | 500 | 33 |

Die vorliegende Erfindung ermöglicht nunmehr auf Basis des hierin offenbarten Wissens -unabhängig von den hierin offenbarten, spezifischen Verbindungen, die nur das Prinzip der Erfindung verdeutlichen bzw. demonstrieren sollen- neue fungizide Wirkstoffe zu identifizieren oder zu optimieren. So können nunmehr beliebige Verbindungen hinsichtlich ihrer Wirkung auf das bislang nicht als Target für Fungizide bekannte Enzym Phosphomannomutase sowohl getestet, fungizide Verbindungen identifiziert und diese in effizienter Weise optimiert werden. Dazu können z.B. die in der vorliegenden Erfindung offenbarten Verfahren verwendet werden.

Die vorliegende Erfindung bezieht sich daher ebenfalls auf die Verwendung von Modulatoren der Phosphomannomutase aus Pilzen, bevorzugt aus pflanzenpathogenen Pilzen als Fungizide.

Die vorliegende Erfindung bezieht sich auch auf Fungizide, die mit Hilfe eines erfindungsgemäßen Verfahrens identifiziert werden.

Wirkstoffe, die mit Hilfe eines erfindungsgemäßen Verfahrens identifiziert werden und/oder die enzymatische Aktivität der Phosphomannomutase inhibieren, können demnach in bestimmten Konzentrationen und Aufwandmengen als Fungizide verwendet werden. Sie lassen sich gegebenenfalls auch als Zwischen- und Vorprodukte für die Optimierung von fungiziden Wirkstoffen und für die Synthese weiterer Wirkstoffe einsetzen.

Erfindungsgemäß können mit diesen Wirkstoffen alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den genannten Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Die Wirkstoffe können in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im Wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen infrage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen infrage: z.B. gebrochene und fraktionierte natürliche Gesteine wie Calcit, Bims, Marmor, Sepiolith, Dolomit sowie synthetische Granulate aus anorganischen und organischen Mehlen sowie Granulate aus organischem Material wie Sägemehl, Kokosnussschalen, Maiskolben und Tabakstängel. Als Emulgier und/oder schaumerzeugende Mittel kommen infrage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen infrage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe als Fungizide können die Aufwandmengen je nach Applikationsart innerhalb eines größeren Bereiches variiert werden. Bei der Behandlung von Pflanzenteilen liegen die Aufwandmengen an Wirkstoff im Allgemeinen zwischen 0,1 und 10 000 g/ha, vorzugsweise zwischen 10 und 1 000 g/ha. Bei der Saatgutbehandlung liegen die Aufwandmengen an Wirkstoff im Allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im Allgemeinen zwischen 0,1 und 10 000 g/ha, vorzugsweise zwischen 1 und 5 000 g/ha.

Die im Folgenden gezeigten Beispiele demonstrieren beispielhaft, wie mit dem erfindungsgemäßen Verfahren Inhibitoren der Phosphomannomutase gefunden werden können und zeigen, dass diese Inhibitoren als Fungizide eingesetzt werden können. Es versteht sich, dass die Beispiele nicht limitierend auszulegen sind.

### Beispiele

### Beispiel 1

### Klonierung, Expression und Reinigung von sec53 bzw. Sec53 aus Saccharomyces cerevisiae

Für die Klonierung von *sec53* bzw. dessen Expression wurde der ORF aus genomischer DNA aus *Saccharomyces cerevisiae* über genspezifische Primer amplifiziert. Die entsprechende DNA, ein Amplikon von 765 bp Länge, wurde in den Vektor pGEX-4T-1 von Pharmacia Biotech zwischenkloniert und anschließend über die durch die Primer eingeführten Schnittstellen *Bam*HI und *Xho*I in den mit Schnittstellen *Bam*HI und *Xho*I geschnittenen Vektor pGEX-4T-1 (Pharmacia Biotech) kloniert. Das resultierende Plasmid pSec53 enthält die vollständige kodierende Sequenz von sec53 in einer N-terminalen Fusion mit dem GST-Tag, das Bestandteil des Vektors ist. Das Sec53 Fusionsprotein besitzt eine berechnete Masse von 53 kD.

Für die heterologe Expression wurde das Plasmid pSec53 so in *E. coli* BL21 (DE3) transformiert, dass der Transformationsansatz direkt als Vorkultur in 50 ml Selektionsmedium diente. Diese Zellen wurden über Nacht bei 37°C inkubiert und anschließend 1:100 in Selektionsmedium (LB-Medium mit 100 µg/ml Ampicillin) verdünnt. Bei einer OD₆₀₀ₙₘ von 0,8 - 1,0 wurde die Temperatur der Zellen auf 18°C gesenkt und mit 1mM IPTG (Endkonzentration) induziert. Die Zellen wurden nach 24 h geerntet. Die Zellpellets können ohne Aktivitätsverlust mehrere Monate bei -80°C gelagert werden. Der Aufschluss erfolgte durch Sonifizieren in Lysepuffer (200 mM KCl, 10 mM Imidazol, 50 mM Tris-HCl, pH 8, 15 % Glycerin). Die Reinigung erfolgte nach dem Standardprotokoll des Herstellers für Glutathion-Sepharose Säulen. Anschließend wurde das gereinigte Protein in Puffer mit Glycerin versetzt (50 mM Tris-HCl pH 8, 5 mM Glutathion, 15 % Glycerin) und bei -80°C gelagert. Aus 250 ml Kulturmedium konnten so etwa 3,5 mg lösliches Protein isoliert werden, das in Verfahren zum Identifizieren von Modulatoren der Phosphomannomutase verwendet werden konnte.

### Beispiel 2

### (A) Identifzierung von Modulatoren der Phosphomannomutase in 384-Well-MTP in einem gekoppelten Assay

Zur Identifizierung von Modulatoren der Phosphomannomutase wurden 384-Well-Mikrotiterplatten von Greiner verwendet.

In die erste Spalte wurde die Negativ-Kontrolle pipettiert. Diese setzte sich zusammen aus 5 µl Testpuffer (2 mM MgCl₂, 100 mM Tris/HCl, pH 7, 1 mM EDTA) mit 5 % DMSO, 20 µl Testpuffer (2 mM MgCl₂, 100 mM Tris/HCl, pH 7, 1 mM EDTA) und 25 µl Mix 1 (100 mM Tris/HCl pH 7, 2 mM MgCl₂, 1 mM EDTA, 1 mM NADP⁺, 0,2 mM D-Glucose-1,6-diphosphat, 0,0125 U Phosphoglucose Isomerase, 0,0125 U Phosphomannose Isomerase, 0,05 U Glucose-6-phosphat Dehydrogenase, 0,05 µg Phosphomannomutase).

In die zweite Spalte wurde die Positiv-Kontrolle pipettiert. Diese setzte sich zusammen aus 5µl Testpuffer mit 5 % DMSO, 20 µl Mix 2 (1 mM D-Mannose-1-phosphat, 2 mM MgCl₂, 100 mM Tris/HCl, pH 7, 1 mM EDTA ) und 25 µl Mix 1 (100 mM Tris/HCl pH 7, 2 mM MgCl₂, 1 mM EDTA, 1 mM NADP⁺, 0,2 mM D-Glucose-1,6-diphosphat, 0,0125 U Phosphoglucose Isomerase, 0,0125 U Phosphomannose Isomerase, 0,05 U Glucose-6-phosphat Dehydrogenase, 0,05 µg Phosphomannomutase).

In die verbleibenden Spalten wurde eine Prüfsubstanz in einer Konzentration von 2 µM in DMSO vorgelegt, wobei zum Verdünnen der Substanz auf ein Volumen von 5 µl der Testpuffer verwendet wurde. Nach Zugabe von 20 µl Mix 2 (1 mM D-Mannose-1-phosphat, 2 mM MgCl₂, 100 mM Tris/HCl, pH 7, 1 mM EDTA ) wurden zum Start der Reaktion 25 µl Mix 1 (100 mM Tris/HCl pH 7, 2 mM MgCl₂, 1 mM EDTA, 1 mM NADP⁺, 0,2 mM D-Glucose-1,6-diphosphat, 0,0125 U Phosphoglucose Isomerase, 0,0125 U Phosphomannose Isomerase, 0,05 U Glucose-6-phosphat Dehydrogenase, 0,05 µg exprimierte und gereinigte Phosphomannomutase) zugegeben. Es folgte eine Inkubation bei Raumtemperatur für 20 Minuten.

Die Messung des während der Reaktion entstehenden NADPH erfolgte durch Bestimmung der absoluten Fluoreszenz in einem für MTP geeigneten Tecan Ultra Fluoreszenzspektrometer.

### Beispiel 3

### Nachweis der fungiziden Wirkung der identifizierten Inhibitoren der Phosphomannomutase

In die Kavitäten von Mikrotiterplatten wurde eine methanolische Lösung des anhand eines erfindungsgemäßen Verfahrens identifizierten Wirkstoffs (Tab. I, Bsp. 2), versetzt mit einem Emulgator, pipettiert. Nachdem das Lösungsmittel abgedampft war, wurden je Kavität 200 µl Potatoe-Dextrose-Medium hinzugefügt. Das Medium wurde vorher mit geeigneten Konzentrationen von Sporen bzw. Mycelen des zu prüfenden Pilzes (siehe Tabelle II) versetzt.

Die resultierenden Konzentrationen des Wirkstoffs betrugen 0,1, 1, 10 und 100 ppm. Die resultierende Konzentration des Emulgators betrug 300 ppm.

Die Platten wurden anschließend auf einem Schüttler bei einer Temperatur von 22°C inkubiert, bis in der unbehandelten Kontrolle ein ausreichendes Wachstum feststellbar war. Die Auswertung erfolgte photometrisch bei einer Wellenlänge von 620 nm. Aus den Messdaten der verschiedenen Konzentrationen wird die Wirkstoffdosis, die zu einer 50%igen Hemmung des Pilzwachstums gegenüber der unbehandelten Kontrolle führt (ED₅₀), berechnet. Verbindung 2 aus Tabelle I zeigte bereits bei der in Tabelle II angegebenen Aufwandmenge eine deutlich fungizide Wirkung.

### Beschreibung der Abbildungen

- Abbildung 1:: Die von der Phosphomannomutase katalysierte Reaktion ist die Isomerisierung von D-Mannose 1-phosphat (**1**) zu D-Mannose 6-phosphat (**2**). Diese von der Phosphomannomutase katalysierte Reaktion beschreibt ihre biologische bzw. enzymatische Aktivität.
- Abbildung 2:: Die beiden Kofaktoren D-Mannose 1,6-bisphosphat (**3**) bzw. D-Glucose 1,6-bisphosphat (**4**) der Phosmannomutase.
- Abbildung 3:: Homologie zwischen Phosphomannomutasen aus verschiedenen Pilzen: (1): *S. cerevisiae* (Sec53), (2): *C. albicans,* (3): *S. pombe,* (4): *N. crassa.* Rahmen geben Bereiche mit einer genau übereinstimmenden Sequenz wieder (Konsensussequenz).
- Abbildung 4:: Heterologe Expression der Phosphomannomutase in *E. coli* BL21 (DE3). Das überexprimierte GST-Fusionsprotein hat eine Größe von 53 kDa. In Spur 1 wurde ein Größenstandard aufgetragen. Die unterschiedliche Dauer der Expression ist in den verschiedenen Spuren nachzuvollziehen: Spuren 2-5: 0 Stunden; Spuren 6-9: 3 Stunden; Spuren 10-13: 24 Stunden.
- Abbildung 5:: Kinetik der Umsetzung von NADP⁺ durch die Phosphomannomutase. In einem Assayvolumen von 50 µl wurden 0,4 mM D-Mannose-1-phosphat, 1 mM NADP⁺, 0,2 mM Glucose, 0,0125 U Phosphoglucose Isomerase, 0,0125 U Phosphomannose Isomerase, 0,05 U Glucose-6-phosphat Dehydrogenase und 0,05 µg Phosphomannomutase eingesetzt. Die verwendeten Proteinkonzentrationen der Phosphomannomutase (PMM) sind der Abbildung zu entnehmen. Die Umsetzung wurde anhand der Fluoreszenz des entstehenden NADPH verfolgt. In der Abbildung ist die relative Fluoreszenz angegeben.

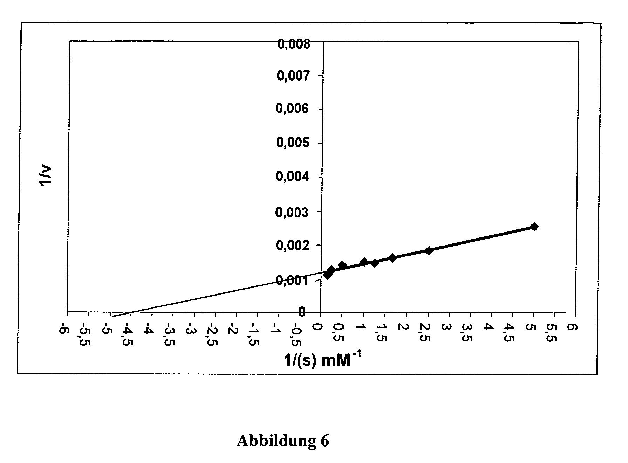

- Abbildung 6:: K_{M}-Wert Bestimmung für D-Mannose-1-phosphat. Darstellung der gemessenen Werte nach Lineweaver und Burk: 1/Vₒ = 1/Vₘₐₓ + 1/S x (K_{M}/Vₘₐₓ), worin Vₒ die anfängliche Reaktionsgeschwindigkeit, Vₘₐₓ die maximal erreichbare Umsatzgeschwindigkeit und S die Substratkonzentarion ist. Vₘₐₓ und K_{M} lassen sich dann als Abszissen- und Ordinatenabschnitt 1/Vₘₐₓ bzw. 1/K_{M} ablesen.

### Literatur

Bernstein M., Hoffmann W., Ammerer G., Schekman R.; RT (1985) Characterization of a gene product (Sec53p) required for protein assembly RT in the yeast endoplasmic reticulum; J. Cell Biol. 101: 2374-2382.

Burda, P. and Aebi, M. (1999) The dolichol pathway of N-linked glycosylation. Biochim. Biophys. Acta 1426(2): 239-257.

Glaser, L.; Kornfeld, S. and Brown, D. H. (1959) Preparation and properties of phosphomannomutase from baker's yeast. Biochim. Biophys. Acta, 33(2): 522-6.

Glaser, L. (1966) Phosphomannomutase from yeast. Methods Enzymol., 8: 183-5.

Herscovics, A. and Orlean, P. (1993). Glycoprotein biosynthesis in yeast. The FASEB Journal 7: 540 -550.

Guha, S. K.; Rose, Z. B. (1985) The synthesis of mannose 1-phosphate in brain. Arch. Biochem. Biophys. 243(1): 168-73.

Hibbs, A. R. and Meyer, D. I. (1988) Secretion in yeast: in vitro analysis of the sec53 mutant. EMBO J. 7(7): 2229-32.

Kepes, F. and Schekman, R. (1988) The yeast SEC53 gene encodes phosphomannomutase. J. Biol. Chem., 263(19): 9155-61.

Kepes, F. (1994) SEC53. Editor(s): Rothblatt, Jonathan; Novick, Peter; Stevens, Tom H. Guideb. Secretory Pathway: 52; Publisher: Oxford University Press.

Martin, A.; Watterson, M. P.; Brown, A. R.; Imtiaz, F.; Winchester, B. G.; Watkin, D. J.; Fleet, G. W. J. (1999) 6R- and 6S-6C-Methylmannose from D-mannuronolactone. Inhibition of phosphoglucomutase and phosphomannomutase: agents for the study of the primary metabolism of mannose. Tetrahedron: Asymmetry 10(2): 355-366.

Oesterhelt, C.; Schnarrenberger. C. and Gross, W. (1997) The reaction mechanism of phosphomannomutase in plants. FEBS Lett., 401(1): 35-37.

## Patentansprüche

1. Verfahren zum Identifizieren von Fungiziden, **dadurch gekennzeichnet, dass** man eine chemische Verbindung in einem Phosphomannomutase-Hemmtest testet.

2. Verfahren gemäß Anspruch 1, **dadurch gekennzeichnet, dass** man
(a) eine Wirtszelle, die eine Phosphomannomutase in ausreichender Menge exprimiert oder ein Polypeptid mit der enzymatischen Aktivität einer Phosphomannomutase mit einer chemischen Verbindung oder einem Gemisch von chemischen Verbindungen unter Bedingungen, die die Interaktion der chemischen Verbindung mit dem Polypeptid erlauben, in Kontakt bringt,
(b) die Aktivität der Phosphomannomutase bei Abwesenheit einer chemischen Verbindung mit der Aktivität der Phosphomannomutase bei Anwesenheit einer chemischen Verbindung oder eines Gemisches von chemischen Verbindungen vergleicht, und
(c) die chemische Verbindung, die die Phosphomannomutase spezifisch inhibiert, bestimmt.

3. Verfahren gemäß Anspruch 1 oder 2, **dadurch gekennzeichnet, dass** man eine Phosphomannomutase aus einem Pilz verwendet.

4. Verfahren gemäß einem der Ansprüche 1 bis 3, **dadurch gekennzeichnet, dass** man in einem weiteren Schritt die fungizide Wirkung der identifizierten Verbindung testet, indem man sie mit einem Pilz in Kontakt bringt.

5. Verfahren gemäß einem der Ansprüche 1 bis 4, **dadurch gekennzeichnet, dass** man die Hemmung der Aktivität der Phosphomannomutase anhand der Menge der durch Säure abgespalteten Phosphatgruppe von D-Mannose-1-phosphat mit einem Phosphatnachweisreagenz bestimmt.

6. Verwendung von Polypeptiden mit der Aktivität einer Phosphomannomutase zum Identifizieren von Fungiziden.

7. Verwendung von Inhibitoren von Polypeptiden mit der Aktivität einer Phosphomannomutase als Fungizide.

8. Verwendung von Inhibitoren eines Polypeptids mit der Aktivität einer Phosphomannomutase, welche durch ein Verfahren gemäß einem der Ansprüche 1 bis 4 identifiziert werden, als Fungizide.

9. Fungizide, welche mittels eines Verfahrens gemäß einem der Ansprüche 1 bis 5 identifiziert werden.
